# FASCICULE DE BREVET EUROPEEN

(11) **EP 4 168 078 B1**
(45) Date de publication et mention de la délivrance du brevet: **07.08.2024**
(21) Numéro de dépôt: 21740138.9
(22) Date de dépôt: 18.06.2021
(51) Int. Cl.: A61M 15/00, A61M 15/08, B65D 83/22, B65D 83/20, B65D 83/38, B05B 11/00, B05B 12/02

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 22.06.2020 FR 2006490
(43) Date de publication de la demande: 26.04.2023
(73) Titulaire: APTAR France SAS, 27110 Le Neubourg (FR)
(72) Inventeur: FABIEN, David, 29290 Saint Renan (FR)
(74) Mandataire: CAPRI
(86) Numéro de dépôt international: PCT/FR2021/051108
(87) Numéro de publication internationale: WO 2021/260305

(56) Documents cités:
- EP-A2- 0 114 617
- US-A- 5 228 586
- US-A1- 2015 320 948

## Description

La présente invention concerne un dispositif de distribution de produit fluide.

De nos jours, l'administration de médicaments puissants potentiellement létaux chez l'homme peut-être une nécessité dans certaines situations. C'est notamment le cas pour le traitement de pathologies particulières ou encore pour des personnes ayant besoin de traitements palliatifs dans des contextes de fin de vie. La manipulation de telles substances requiert une grande prudence et des dispositifs d'administration extrêmement sûrs, pour éviter les risques de surdose, qui peuvent survenir en cas d'administration rapprochée de plusieurs doses consécutives.

Le document US5228586 décrit un dispositif tel que décrit dans le préambule de la revendication 1. Les documents EP0114617 et US2015320948 décrivent d'autres dispositifs de l'état de la technique.

La présente invention a pour but de fournir un dispositif de distribution de produit fluide qui ne reproduit pas les inconvénients susmentionnés.

La présente invention a notamment pour but de fournir un dispositif de distribution de produit fluide qui soit sécurisé et sécurisant pour l'utilisateur, notamment pour éviter les risques d'overdose.

La présente invention a également pour but de fournir un dispositif de distribution de produit fluide qui soit bloqué pendant un temps prédéterminable entre deux actionnements successifs.

La présente invention a aussi pour but de fournir un dispositif de distribution de produit fluide qui soit robuste et fiable d'utilisation.

La présente invention a également pour but de fournir un dispositif de distribution de produit fluide qui soit simple et peu coûteux à fabriquer et à assembler.

La présente invention a donc pour objet un dispositif de distribution de produit fluide comportant:
- un distributeur de produit fluide comportant un réservoir contenant du produit fluide et un organe de distribution, tel qu'une pompe ou une valve, monté sur ledit réservoir,
- un corps interne comportant un cylindre creux recevant ledit distributeur,
- un poussoir pourvu d'un orifice de distribution, monté axialement déplaçable sur ledit corps interne entre une position de repos et une position d'actionnement, le déplacement axial dudit poussoir de sa position de repos vers sa position d'actionnement actionnant ledit organe de distribution pour distribuer une dose de produit fluide à travers ledit orifice de distribution,
- un moteur,
- un module de commande électronique,
- une roue d'engrenage reliée audit moteur et entrainée en rotation par ledit moteur, et
- une bague de verrouillage montée rotative sur ledit corps interne entre une position de verrouillage et une position de libération, un ressort sollicitant ladite bague de verrouillage vers sa position de verrouillage, ladite bague de verrouillage étant déplacée de sa position de verrouillage vers sa position de libération par ladite roue d'engrenage, ladite bague de verrouillage coopérant en position de verrouillage avec ledit poussoir pour empêcher son déplacement axial et coopérant en position de libération avec ledit poussoir pour permettre son déplacement axial vers sa position d'actionnement.

Avantageusement, le dispositif comporte un corps externe fixé audit corps interne et contenant ledit moteur et ledit module de commande électronique,

Avantageusement, le dispositif comporte un bouton de commande pour déclencher l'actionnement dudit moteur.

Avantageusement, ledit poussoir comporte au moins un montant axial et ladite bague de verrouillage comporte au moins au moins une projection radiale.

Avantageusement, en position de verrouillage de ladite bague de verrouillage, ladite au moins une projection radiale coopère avec ledit au moins un montant axial, pour empêcher le déplacement axial dudit poussoir vers sa position d'actionnement, et, en position de libération de ladite bague de verrouillage, ladite au moins une projection radiale est décalée angulairement dudit au moins un montant axial, permettant ainsi un déplacement axial dudit poussoir vers sa position d'actionnement.

Avantageusement, le dispositif comporte quatre projections radiales et quatre montants axiaux, chaque projection radiale coopérant avec un montant axial respectif pour bloquer le déplacement axial dudit poussoir en position de verrouillage de ladite bague de verrouillage.

Avantageusement, ladite bague de verrouillage comporte une lame axialement flexible pourvue d'une dent saillante axialement vers le haut.

Avantageusement, ledit corps interne, ou un élément solidaire dudit corps interne, tel qu'un couvercle, comporte un profil interne, comportant une première fenêtre et une seconde fenêtre, séparée de ladite première fenêtre par un montant radial.

Avantageusement, ledit poussoir comporte un doigt axial s'étendant axialement vers le bas.

Avantageusement, en position de verrouillage de ladite bague de verrouillage, ladite dent est disposée dans ladite première fenêtre, le déplacement de ladite bague de verrouillage vers sa position de libération déformant axialement ladite lame flexible pour permettre à ladite dent de passer dans la seconde fenêtre, derrière ledit montant radial, ledit montant radial coopérant alors avec ladite dent pour empêcher ladite bague de verrouillage de revenir en position de verrouillage sous l'effet dudit ressort.

Avantageusement, lors du déplacement axial dudit poussoir de sa position de repos vers sa position d'actionnement, ledit doigt axial coopère avec ladite dent en la poussant axialement vers le bas pour la désengager dudit montant radial, permettant ainsi à ladite bague de verrouillage de revenir vers sa position de verrouillage.

Avantageusement, tant que ledit poussoir est déplacé hors de sa position de repos, ledit poussoir bloque ladite bague de verrouillage dans une position intermédiaire entre sa position de libération et sa position de verrouillage.

Avantageusement, en fin d'actionnement, lorsque ledit poussoir revient dans sa position de repos, ladite bague de verrouillage revient automatiquement dans sa position de verrouillage sous l'effet dudit ressort.

Avantageusement, ledit module de commande électronique comporte des moyens de temporisation pour empêcher après chaque actionnement du distributeur le déplacement de ladite bague de verrouillage de sa position de verrouillage vers sa position de libération pendant un temps prédéterminable.

Avantageusement, lesdits moyens de temporisation bloquent un bouton de commande du dispositif et/ou ledit moteur.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
la figure 1 est une vue schématique en perspective éclatée d'un dispositif de distribution selon un mode de réalisation avantageux,
la figure 2 est une vue schématique en perspective éclatée illustrant l'assemblage du distributeur de produit fluide dans le dispositif,
la figure 3 est une vue schématique en perspective de côté du dispositif de la figure 1, illustrant l'étape de déverrouillage du dispositif,
la figure 4 est une vue similaire à celle de la figure 3, illustrant l'étape d'actionnement du distributeur de produit fluide,
la figure 5 est une vue schématique en perspective de la bague de verrouillage, selon un mode de réalisation avantageux,
la figure 6 est une vue schématique en perspective de détail d'une partie de la bague de verrouillage de la figure 5,
la figure 7 est une vue schématique similaire à celle de la figure 5, selon un autre angle de vue,
la figure 8 est une vue schématique en perspective de dessous du poussoir,
la figure 9 est une vue schématique en perspective du couvercle, selon un mode de réalisation avantageux,
la figure 10 est une vue schématique en perspective de la roue d'engrenage, selon un mode de réalisation avantageux,
la figure 11 est une vue schématique partielle en perspective montrant le déplacement axial du poussoir lors de l'actionnement,
la figure 12 est une vue schématique similaire à celle de la figure 11, montrant le déplacement en rotation de la bague de verrouillage lors du déverrouillage,
la figure 13 est une vue schématique en perspective découpée montrant la bague de verrouillage sollicitée vers sa position de verrouillage,
la figure 14 est une vue schématique en section transversale montrant la coopération entre la bague de verrouillage et la roue d'engrenage,
les figures 15 et 16 sont des vues schématiques en perspective découpée qui montrent le poussoir, la bague de verrouillage et la roue d'engrenage, respectivement en position de verrouillage et en position de libération,
les figures 17 et 18 sont des vues schématiques en perspective découpée qui montrent la bague de verrouillage, respectivement en position de verrouillage et en position de libération,
la figure 19 est une vue schématique de détail en perspective découpée montrant la patte flexible de la bague de verrouillage en position de verrouillage,
les figures 20 et 21 sont des vues schématiques en perspective découpée qui montrent la coopération entre la bague de verrouillage et la roue d'engrenage pendant un cycle de déverrouillage,
les figures 22 à 26 sont des vues schématiques de détail en perspective découpée qui montrent la patte flexible au cours d'un cycle d'actionnement du dispositif,
les figures 27 et 28 sont des vues schématiques en section transversale horizontale qui montrent la bague de verrouillage respectivement en position de libération et en position intermédiaire en cours d'actionnement du dispositif,
la figure 29 est une vue schématique en section transversale verticale du poussoir avant qu'il ne revienne en position de repos, et
les figures 30 et 31 sont des vues schématiques partielles en perspective découpée, montrant la bague de verrouillage revenant en position de verrouillage en fin d'actionnement, lorsque le poussoir revient en position de repos.

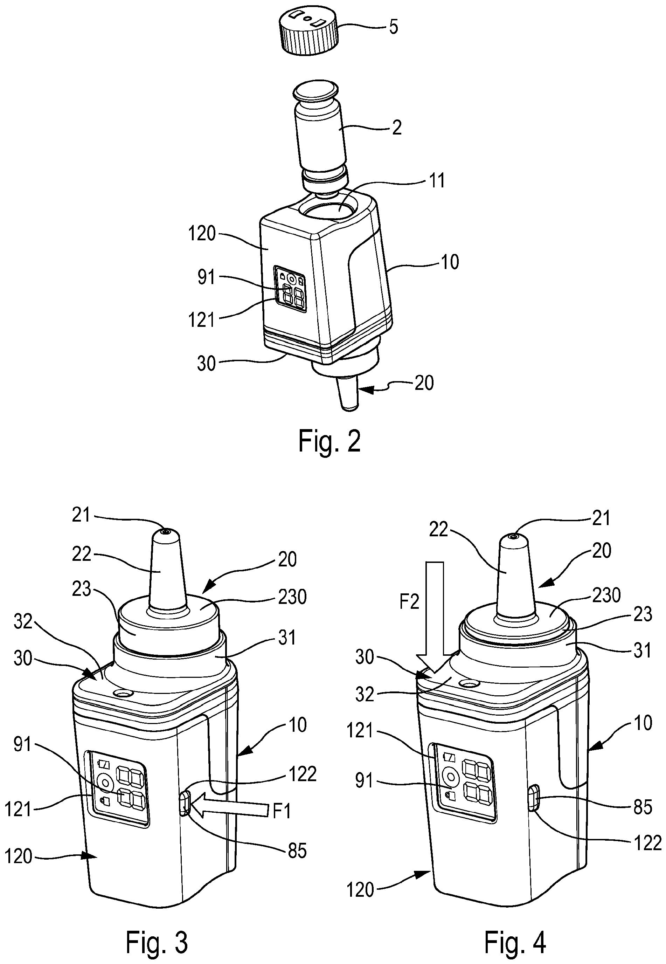

Les termes "axial", "radial", "horizontal" et "vertical" se réfèrent à l'axe central longitudinal du dispositif. Les termes "haut", "bas", "supérieur" et "inférieur" se réfèrent à la position droite du dispositif représentée sur les figures 3 et 4.

La présente invention a principalement pour objet un dispositif qui autorise/interdit la délivrance de dose en bloquant/libérant l'actionnement d'un poussoir permettant l'actionnement d'un distributeur de produit fluide.

Le dispositif de distribution de produit fluide représenté sur les figures comporte un distributeur de produit fluide 1, avantageusement du type standard. Ce distributeur 1 comporte un réservoir 2 contenant le produit fluide et un organe de distribution 3, tel qu'une pompe ou une valve, dont une partie est axialement déplaçable par rapport au réservoir 2. L'organe de distribution 3 est fixé sur le réservoir 2 au moyen d'une bague de fixation 4. L'organe de distribution 3 est actionné lorsqu'un poussoir est déplacée axialement vers le bas par rapport au réservoir 2. Généralement, lorsque le réservoir 2 ne contient pas de gaz propulseur, on utilise une pompe doseuse, et lorsque le réservoir 2 contient du gaz propulseur, on utilise une valve doseuse. Ces deux types d'organe de distribution sont bien connus de l'homme du métier et puisque cet organe de distribution n'intervient pas directement dans la présente invention, il ne sera pas décrit plus en détail ci-après.

Le dispositif de distribution de produit fluide comporte également un corps interne 10, recevant le distributeur 1, et un corps externe 120, fixé au corps interne 10 et contenant un module de commande électronique 90.

Le corps interne 10 comporte un cylindre creux 11 ouvert axialement du côté inférieur, avec des moyens d'accouplement, avantageusement réalisés sous la forme d'un filetage 12 sur la surface extérieure de l'ouverture inférieure. Le cylindre creux 11 est destiné à recevoir le réservoir 2 du distributeur 1. Un capot 5 est prévu pour fixer le distributeur 1 dans le corps interne 10. Ce capot 5 comporte des moyens d'accouplement complémentaires, avantageusement réalisés sous la forme d'un filetage interne 6, adaptés à coopérer avec les moyens d'accouplement du corps interne 10. Ainsi, pour assembler le distributeur 1 dans le dispositif, on insère le réservoir 2 à l'intérieur du cylindre creux 11 du corps interne 10. Le capot 5 est alors vissé sur ledit corps interne 10. Ce procédé d'assemblage est représenté sur la figure 2. Cette mise en oeuvre permet de facilement remplacer un distributeur 1 dont le réservoir est vide par un distributeur plein. Le dispositif, et notamment le module de commande électronique 90 peuvent alors être réutilisable au lieu d'être jeté.

Du côté supérieur, le corps interne 10 comporte un plateau 13 et une extension axiale creuse 14, s'étendant axialement vers le haut à partir dudit plateau 13. Cette extension axiale creuse 14 est de dimension radiale réduite par rapport au diamètre interne dudit cylindre creux 11. Elle peut être moulés d'une pièce avec le corps interne 10, ou fabriquée séparément puis fixée audit corps interne 10 d'une quelconque manière appropriée. Lorsque le distributeur 1 est disposé dans le corps interne 10, au moins une partie de l'organe de distribution 3 traverse ladite extension axiale creuse 14.

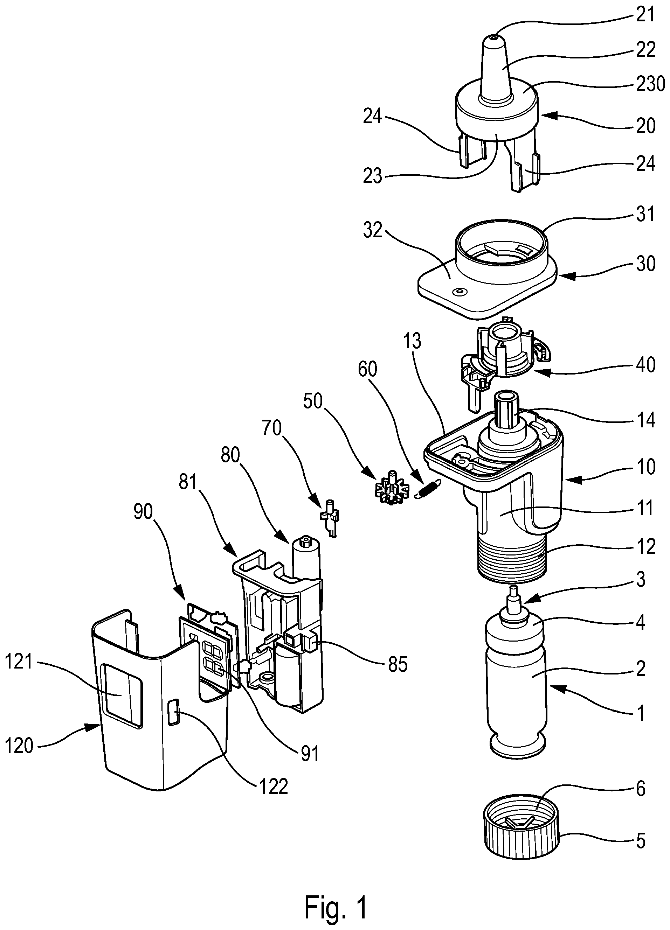

Le corps externe 120 est creux et peut avoir une forme extérieure quelconque. Dans l'exemple représenté, le corps externe 120 est de forme environ rectangulaire qui correspond partiellement à la forme dudit plateau 13 du corps interne 10. Le corps externe 120 se fixe d'une manière appropriée sur le corps interne 10, par exemple par encliquetage. Avantageusement, le corps externe 120 comporte une ou plusieurs fenêtres 121, par exemple une fenêtre comme représenté sur les figures 1 à 4, permettant de visualiser un écran 91 du module de commande électronique 90. Cet écran peut afficher des informations, comme par exemple des instructions d'utilisation, des informations de charge de batterie, etc.

Le corps externe 120 comporte aussi une ouverture 122 pour recevoir un bouton de commande 85. En variante, le bouton de commande pourrait être remplacé par une zone de commande sur l'écran 91 ou sur un autre afficheur visible dans une autre fenêtre. Cette zone de commande pourrait intégrer des moyens de détection d'empreinte digitale pour n'autoriser l'actionnement du dispositif qu'à la ou aux personnes autorisées, et empêcher ainsi tout actionnement accidentel, par exemple par des enfants. D'autres moyens de reconnaissance pourraient être envisagés, tels qu'une reconnaissance faciale. Le bouton ou la zone de commande pourrait aussi être remplacée par d'autres moyen: commande vocale, déverrouillage à distance par un tiers (par exemple un médecin) ou commande gérée par un timer ou un logiciel du module de commande électronique.

Un couvercle 30 est prévu pour se fixer sur le plateau 13 du corps interne 10, ledit couvercle 30 comportant un cylindre creux 31 et une bride radiale 32. Le cylindre creux 31 comporte un profil interne 35, comportant une première fenêtre 350 et une seconde fenêtre 351, séparée de la première fenêtre 350 par un montant radial 352. En variante, le profil 35 pourrait être prévu sur le corps interne 10.

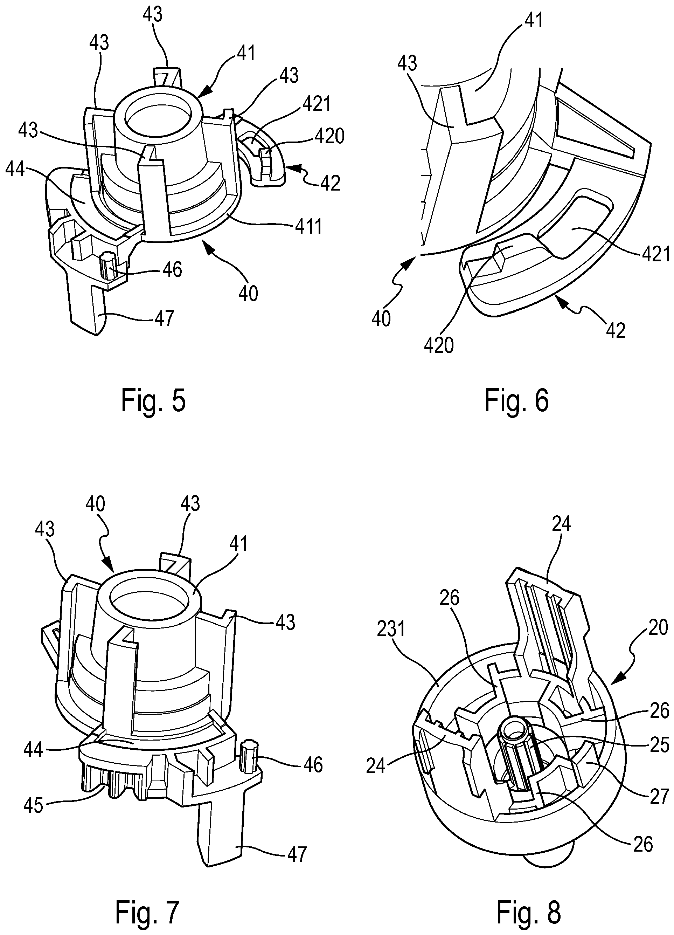

Un poussoir 20, pourvu d'un orifice de distribution 21, est assemblé dans le cylindre creux 31 dudit couvercle 30, en étant axialement déplaçable par rapport au corps interne 10 entre une position de repos et une position d'actionnement, dans laquelle le poussoir 20 est déplacé axialement vers le bas par rapport au corps interne 10. Le poussoir comporte avantageusement un embout nasal 22 qui se termine à son extrémité supérieure par l'orifice de distribution 21 et qui comporte du côté inférieur une jupe axiale 23 pourvue d'une surface d'appui radiale 230 sur laquelle l'utilisateur peut appuyer pour actionner le distributeur 1. Au moins une patte axiale 24 s'étend axialement vers le bas à partir du bord radial inférieur 231 de ladite jupe axiale 23, pour fixer, par exemple par encliquetage, le poussoir 20 dans le cylindre creux 31 du couvercle 30. Dans l'exemple de la figure 8, il y a deux pattes axiales 24 diamétralement opposées. Un doigt axial 27 s'étend aussi axialement vers le bas à partir du bord radial inférieur 231 de ladite jupe axiale 23, dont la fonction sera décrite ultérieurement. Dans l'exemple de la figure 8, ce doigt axial 27 est disposé à 90° de chacune des deux pattes axiales 24 diamétralement opposées.

A l'intérieur, le poussoir 20 comporte un tube creux 25 qui va coopérer avec l'organe de distribution 3 et qui débouche dans l'orifice de distribution 21, avec avantageusement un profil de pulvérisation prévu juste en amont dudit orifice de distribution pour générer un spray. Au moins un montant axial 26 est prévu sous ladite surface d'appui radiale 23. Dans l'exemple de la figure 8, il y a quatre montants axiaux 26 (dont trois sont visibles), répartis autour de la périphérie. La fonction de ces montants axiaux sera décrite ultérieurement.

Le dispositif comporte en outre une bague de verrouillage 40, une roue d'engrenage 50 coopérant avec ladite bague de verrouillage 40, un ressort 60 pour ladite bague de verrouillage 40, et une roue de moteur 70 associée à un moteur 80, ladite roue de moteur 70 coopérant avec ladite roue d'engrenage 50.

La bague de verrouillage 40, la roue d'engrenage 50, le ressort 60 et la roue de moteur 70 sont disposés sur le plateau 13 du corps interne 10 et maintenus en place par le couvercle 30.

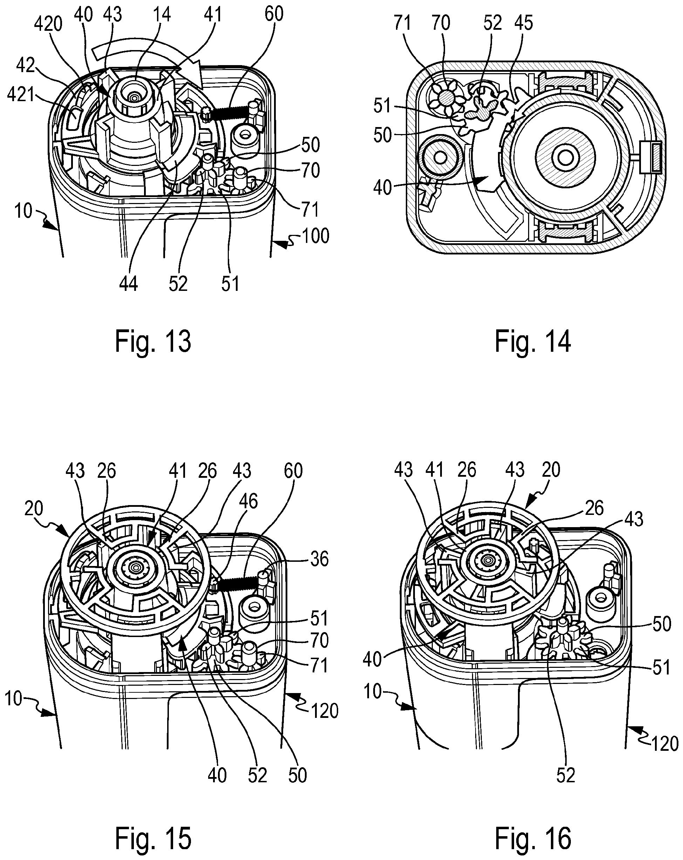

La bague de verrouillage 40 est montée rotative autour de ladite extension axiale 14 du corps interne 10 entre une position de verrouillage et une position de libération. Le ressort 60 sollicite la bague de verrouillage vers sa position de verrouillage. Il est avantageusement fixé d'une part sur un plot 46 de la bague de verrouillage 40 et d'autre part sur un second plot 36 prévu sur le plateau 13 du corps interne 10, comme visible sur les figures 13 et 15. La bague de verrouillage 40 coopère avec le poussoir 20 pour sélectivement bloquer ou permettre le déplacement axial dudit poussoir 20.

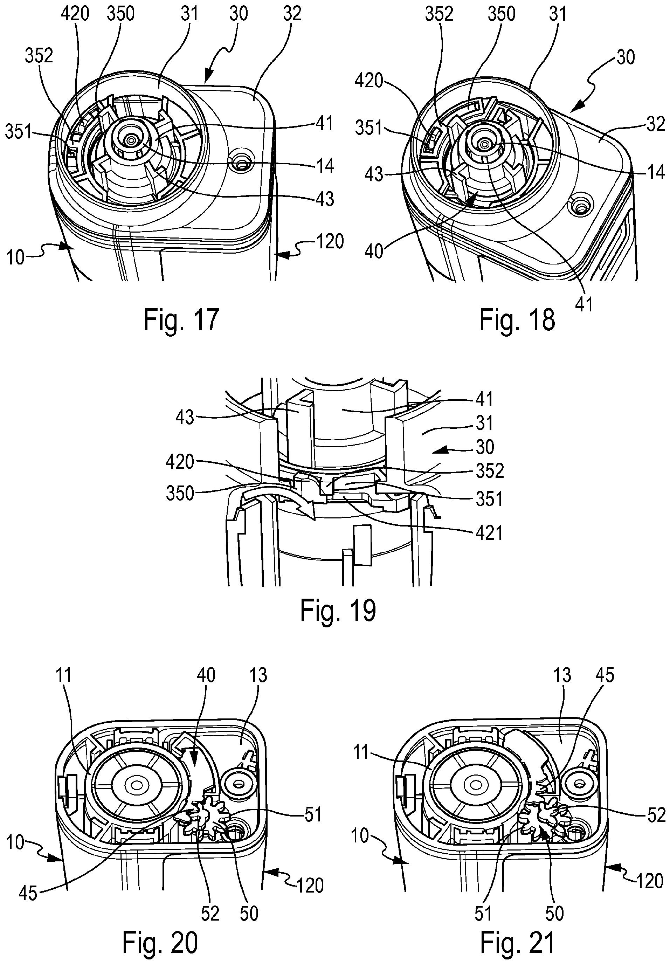

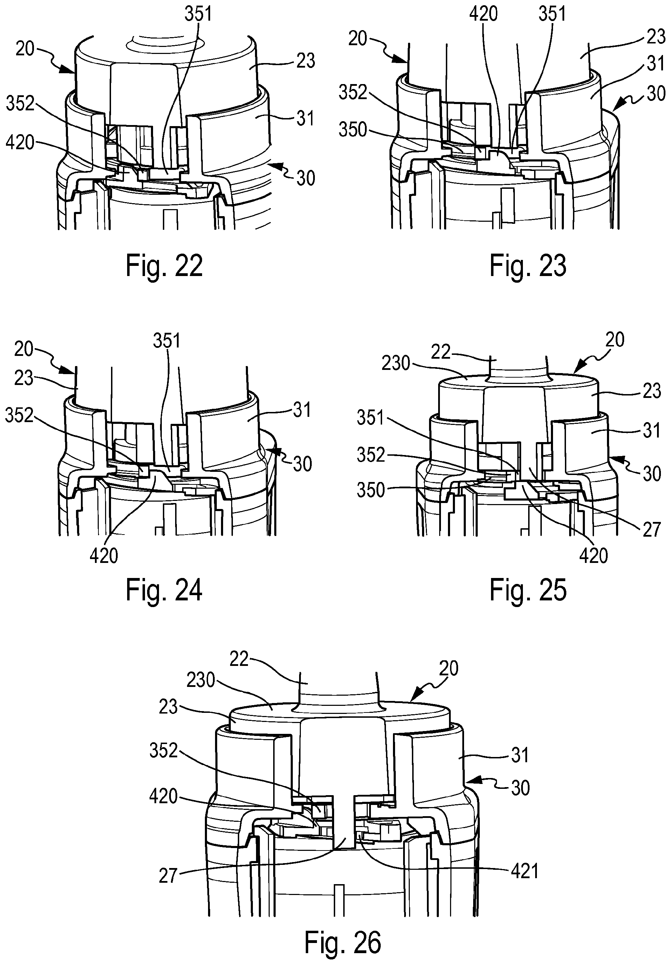

La bague de verrouillage 40 comporte un manchon creux 41 pourvu au niveau d'un bord axial inférieur 411 d'une lame axialement flexible 42 s'étendant coaxialement radialement à l'extérieur dudit manchon creux 41. Cette lame axialement flexible 42 comporte une dent 420 saillante axialement vers le haut, et une ouverture 421 disposée derrière ladite dent 420. En position de verrouillage de la bague de verrouillage, la dent 420 est disposée dans la première fenêtre 350 du couvercle 30, comme visible sur les figures 17, 19 et 22. Lorsque la bague de verrouillage 40 est déplacée vers sa position de libération, la lame axialement flexible 42 se déforme axialement vers le bas, comme indiqué par la flèche sur la figure 19, pour permettre à la dent 420 de passer sous le montant radial 352 du couvercle 30, pour venir se positionner dans la seconde fenêtre 351, comme visible sur la figure 18. Avantageusement, pendant le cycle de déverrouillage, la rotation du moteur 80 transmise par la roue d'engrenage 50 créée une sur-course angulaire de la bague de verrouillage 40 et donc un décalage entre la dent 420 et le montant radial 352, comme visible sur la figure 23. Ceci permet de garantir que la dent 420 va venir s'encliqueter derrière le montant radial 352 lorsque le moteur 80 n'exerce plus de couple, alors que la seconde denture 52 de la roue d'engrenage 50 n'est plus en prise avec les dents 45 de la bague de verrouillage 40. Ceci permet de garantir un fonctionnement fiable malgré les tolérances de fabrications des différentes pièces. Du fait que le ressort 60 sollicite la bague de verrouillage 40 vers sa position de verrouillage, dès que la bague de verrouillage 40 arrive en position de libération, le ressort va tenter de la ramener en position de verrouillage, mais ce déplacement va être bloqué par le montant radial 352 contre lequel la dent 420 de la lame flexible 42 va buter, comme visible sur la figure 24.

La bague de verrouillage 40 comporte au moins une projection radiale 43 s'étend radialement vers l'extérieur à partir dudit manchon creux 41. Dans l'exemple des figures, il y a quatre projections radiales 43 réparties autour de la périphérie du manchon creux 41. En position de verrouillage, ces projections radiales 43 coopèrent avec les montants axiaux 26 du poussoir 20, pour empêcher le déplacement axial du poussoir vers sa position d'actionnement. Au contraire, en position de libération, chaque projection radiale 43 est décalée angulairement par rapport au montant axial 26 respectif, permettant ainsi un déplacement axial dudit poussoir 20 vers sa position d'actionnement. La mise en oeuvre représentée sur les dessins, avec quatre projections radiales 43 coopérant avec quatre montants axiaux 26, visible sur la figure 15, rend le dispositif particulièrement robuste et empêche tout actionnement non autorisé du dispositif.

Du côté opposé à la lame flexible 42, la bague de verrouillage 40 comporte un plat 44 qui supporte des dents 45 qui coopèrent avec la roue d'engrenage 50 lorsque celle-ci déplace la bague de verrouillage 40 vers sa position de libération. Le plat 44 supporte aussi avantageusement le plot 46 fixé au ressort 60. Avantageusement, la bague de verrouillage 40 comporte des moyens de détection adaptés à coopérer avec le module de commande électronique 90 pour l'informer de sa position. Dans l'exemple représenté, ces moyens de détection comprennent une extension axiale 47 s'étendant axialement vers le bas à partir dudit plat 44.

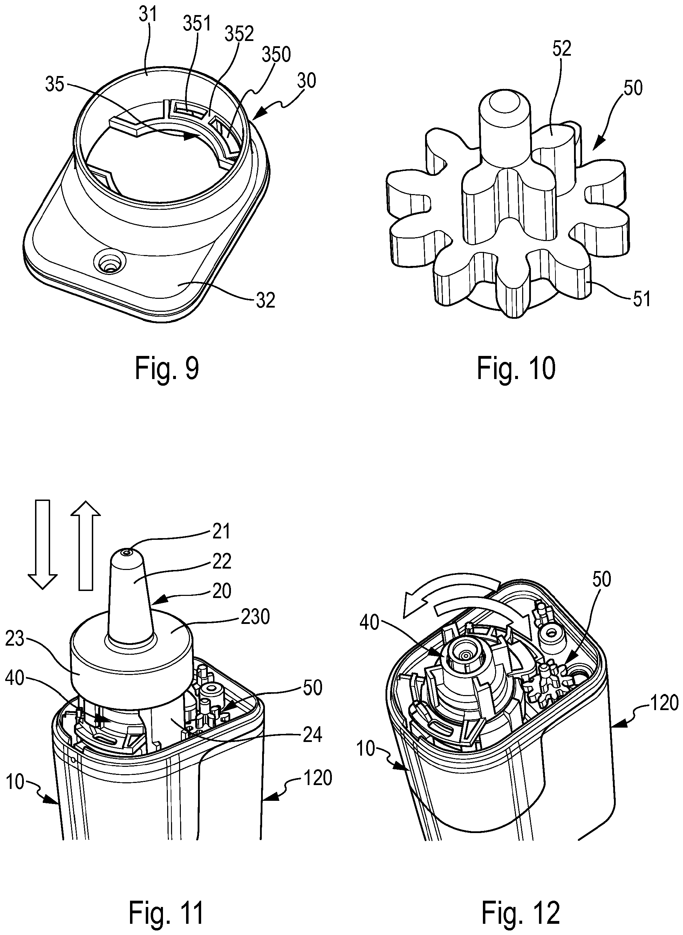

La roue d'engrenage 50 comporte une première denture 51 comportant plusieurs dents réparties sur toute la périphérie de la roue d'engrenage 50. Axialement au-dessus de la première denture 51, la roue d'engrenage 50 comporte une seconde denture 52. Celle-ci ne comporte des dents que sur une partie de la périphérie. Dans l'exemple représenté sur la figure 10, la seconde denture 52 comporte trois dents. Cette seconde denture 52 coopère avec les dents 45 de la bague de verrouillage 40, pour la faire tourner de sa position de verrouillage vers sa position de libération, comme visible sur les figures 20 et 21.

La roue de moteur 70 comporte une denture 71 coopérant avec la première denture 51 de la roue d'engrenage 50. Ainsi, lorsque le moteur 80 fait tourner la roue de moteur 70 dans un sens de rotation, celle-ci fait tourner la roue d'engrenage 50, ce qui entraine la bague de verrouillage 40 de sa position de verrouillage vers sa position de libération.

Un module de commande électronique 90 est fixé sur un corps de support 81, lui-même fixé sur le corps interne 10, l'ensemble étant reçu à l'intérieur dudit corps externe 120. Ce module de commande électronique 90 commande un moteur 80, également fixé au corps de support 81, et qui coopère avec la roue de moteur 70 pour la faire tourner.

Le moteur 80 peut être un moteur à engrenage 3V à courant continu adapté à faire tourner un arbre du moteur connecté à la roue de moteur 70. Ce moteur peut être alimenté d'une quelconque manière appropriée, par exemple au moyen de batteries ou d'accumulateurs, rechargeables ou non. De préférence, lorsque le moteur 80 est commandé, il fait tourner la roue de moteur 70 de telle sorte que la roue d'engrenage 50 effectue un tour complet. Ceci provoque d'abord la rotation de la bague de verrouillage 40 de sa position de verrouillage vers sa position de libération, puis désengage la seconde denture 52 de la roue d'engrenage 50 des dents 45 de la bague de verrouillage 40. Ainsi, une fois que la bague de verrouillage 40 est en position de libération, elle n'est plus connectée au moteur 80.

Le module de commande électronique 90 comporte des éléments électroniques appropriés, tels que notamment un microprocesseur, pour faire fonctionner le dispositif, notamment le moteur 80 et l'écran 91. Avantageusement, le module de commande électronique 90 comporte des switchs ou commutateurs (non représentés) pour détecter le déplacement et/ou la position de la bague de verrouillage 40, notamment de son extension axiale 47. On peut ainsi détecter diverses phases d'actionnement, en particulier le retour de la bague de verrouillage 40 dans sa position de verrouillage, après actionnement du distributeur 1 et donc distribution d'une dose de produit fluide. Ces informations peuvent être utilisées pour bloquer le dispositif pendant un temps prédéterminé.

Ainsi, le module de commande électronique 90 peut comporter des moyens de temporisation pour n'autoriser un nouvel actionnement qu'après l'expiration d'un délai prédéterminable. Ces moyens de temporisation peuvent notamment comprendre l'horloge interne du microprocesseur. Eventuellement, il est possible de lui adjoindre un composant horloge en temps réel. Ce blocage temporaire agit de préférence sur la commande du moteur 80, empêchant ainsi celui-ci de tourner pour déplacer la bague de verrouillage 40 de sa position de verrouillage vers sa position de libération. En variante, c'est le bouton de commande 85 qui peut être désactivé ou bloqué pendant un temps prédéterminable. Avantageusement, seule des personnes autorisées, tels que le personnel médical, peuvent modifier ledit temps de blocage en ayant accès au module de commande électronique ou via l'écran 91. Avantageusement, l'écran 91 indique combien de temps il reste avant de pouvoir et/ou devoir prendre la prochaine dose. Eventuellement, un signal sonore et/ou visuel peut également être prévu si l'utilisateur appuie tout de même sur le bouton de commande pour tenter de déverrouiller le dispositif.

Le fonctionnement du dispositif représenté sur les dessins va maintenant être décrit plus en détail.

Dans un cycle d'actionnement normal, l'utilisateur prend le dispositif dans sa main en position de repos, représentée sur la figure 3. Dans cette position, il ne peut pas déplacer le poussoir 20 axialement vers le bas, car celui-ci est bloqué par la bague de verrouillage 40 qui est en position de verrouillage.

Pour actionner le dispositif, l'utilisateur doit d'abord réaliser une commande du module de commande électronique 90, pour déplacer la bague de verrouillage 40 de sa position de verrouillage vers sa position de libération. Pour ce faire, il appuie sur le bouton de commande 85, dans l'exemple des dessins, selon la flèche F1 sur la figure 3. Cet appui va faire tourner le moteur 80 et donc la roue de moteur 70, ce qui va faire tourner la roue d'engrenage 50 et donc la bague de verrouillage 40 depuis sa position de verrouillage, visible sur les figures 15, 17, 19 et 22, vers sa position de libération, visible sur les figures 16, 18 et 23. Ce déplacement de la roue de verrouillage 60 va charger le ressort 60, celui-ci agissant pour ramener la bague de verrouillage 40 vers sa position de verrouillage. Mais comme la bague de verrouillage 40 est bloquée en position de libération par la dent 420 qui bute contre le montant radial 352 du couvercle 30, elle ne peut pas revenir vers sa position de verrouillage sous l'effet de la force exercée par le ressort 60.

L'utilisateur peut alors exercer un effort d'actionnement axial sur le poussoir 20 pour le déplacer axialement vers le bas selon la flèche F2 sur la figure 4. Ceci va actionner l'organe de distribution 3 et expulser une dose de produit fluide à travers l'orifice de distribution 21. L'actionnement du moteur 80 a fait tourner la roue d'engrenage 50 d'un tour complet, de sorte qu'en position de libération, la bague de verrouillage 40 n'est plus reliée au moteur 80, puisque la seconde denture 52 de la roue d'engrenage 50 n'est plus connectée aux dents 45 de la bague de verrouillage 40. En variante, la roue d'engrenage 50 pourrait ne pas faire un tour complet à chaque actionnement du moteur 80 mais une course angulaire quelconque, par exemple un demi-tour. Dans ce cas, le nombre de dents 45 de la bague de verrouillage 40 et le nombre de dents de la seconde denture 52 de la roue d'engrenage seraient adaptées en conséquence.

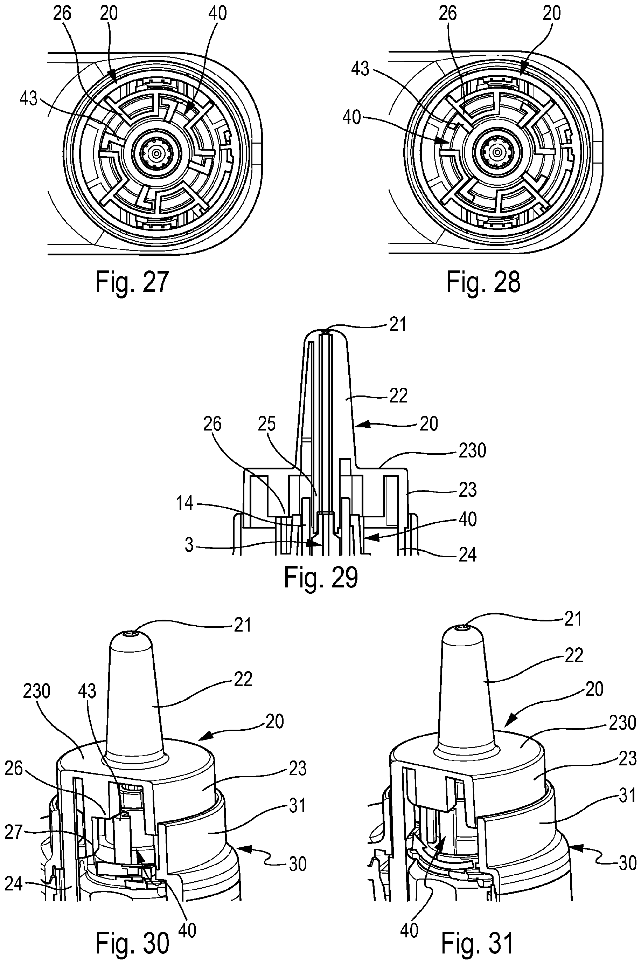

Dès le début de l'actionnement, le poussoir 20 descend axialement par rapport à la bague de verrouillage 40, avec le doigt axial 27 du poussoir qui va coopérer avec la dent 420 de la lame flexible 42. Comme visible sur la figure 25, le doigt axial 27 va pousser la dent 420 axialement vers le bas, de sorte que celle-ci va se désengager du montant radial 352. Lorsque la dent 420 n'est plus bloquée par le montant radial 352, la bague de verrouillage 40 est sollicitée vers sa position de verrouillage par le ressort 60. Elle ne peut toutefois pas revenir dans cette position de verrouillage, car les projections radiales 43 de la bague de verrouillage 40 restent bloqués contre les montants radiaux 26 du poussoir 20, dans une position intermédiaire visible sur la figure 28. Ce n'est que lorsque le poussoir 20 sera revenu dans sa position de repos que la bague de verrouillage 40 pourra automatiquement revenir dans sa position de verrouillage sous l'effet du ressort 60. De préférence, le désengagement de la dent 420 du montant radial 352 se produit au début de la course d'actionnement du poussoir 20, ce qui garantit un verrouillage même en cas de course d'actionnement incomplète. Il n'est donc pas possible de distribuer plusieurs doses partielles consécutives. Après désengagement de la dent 420, lorsque la bague de verrouillage 40 est dans la position intermédiaire de la figure 28, le poussoir 20 peut poursuivre sa course d'actionnement complète jusqu'à sa position d'actionnement, avec le doigt axial 27 qui traverse l'ouverture 421 de la lame flexible 42, comme visible sur la figure 26.

Après l'actionnement, l'utilisateur relâche sa pression sur le poussoir 20, qui va être ramené vers sa position de repos par l'organe de distribution 3; en particulier son ressort de rappel (non représenté).

Le dispositif est alors revenu en position de repos, et un prochain actionnement ne sera possible qu'après l'expiration du délai de blocage prédéterminé par le module de commande électronique 90.

Bien que la présente invention ait été décrite en référence à un mode de réalisation avantageux, il est entendu que l'homme du métier peut y apporter diverses modifications sans sortir du cadre de la présente invention tel que défini par les revendications annexées.

## Revendications

1. Dispositif de distribution de produit fluide comportant:
- un distributeur de produit fluide (1) comportant un réservoir (2) contenant du produit fluide et un organe de distribution (3), tel qu'une pompe ou une valve, monté sur ledit réservoir (2),
- un corps interne (10) comportant un cylindre creux (11) recevant ledit distributeur (1),
- un poussoir (20) pourvu d'un orifice de distribution (21), monté axialement déplaçable sur ledit corps interne (10) entre une position de repos et une position d'actionnement, le déplacement axial dudit poussoir (20) de sa position de repos vers sa position d'actionnement actionnant ledit organe de distribution (3) pour distribuer une dose de produit fluide à travers ledit orifice de distribution (21),
**caractérisé en ce que** ledit dispositif comporte:
- un moteur (80),
- un module de commande électronique (90),
- une roue d'engrenage (50) reliée audit moteur (80) et entrainée en rotation par ledit moteur (80), et
- une bague de verrouillage (40) montée rotative sur ledit corps interne (10) entre une position de verrouillage et une position de libération, un ressort (60) sollicitant ladite bague de verrouillage (40) vers sa position de verrouillage, ladite bague de verrouillage (40) étant déplacée de sa position de verrouillage vers sa position de libération par ladite roue d'engrenage (50), ladite bague de verrouillage (40) coopérant en position de verrouillage avec ledit poussoir (20) pour empêcher son déplacement axial et coopérant en position de libération avec ledit poussoir (20) pour permettre son déplacement axial vers sa position d'actionnement.

2. Dispositif selon la revendication 1, comportant un corps externe (120) fixé audit corps interne (10) et contenant ledit moteur (80) et ledit module de commande électronique (90),

3. Dispositif selon la revendication 1 ou 2, comportant un bouton de commande (85) pour déclencher l'actionnement dudit moteur (80).

4. Dispositif selon l'une quelconque des revendications précédentes, dans lequel ledit poussoir (20) comporte au moins un montant axial (26) et ladite bague de verrouillage (40) comporte au moins au moins une projection radiale (43).

5. Dispositif selon la revendication 4, dans lequel, en position de verrouillage de ladite bague de verrouillage (40), ladite au moins une projection radiale (43) coopère avec ledit au moins un montant axial (26), pour empêcher le déplacement axial dudit poussoir (20) vers sa position d'actionnement, et, en position de libération de ladite bague de verrouillage (40), ladite au moins une projection radiale (43) est décalée angulairement dudit au moins un montant axial (26), permettant ainsi un déplacement axial dudit poussoir (20) vers sa position d'actionnement.

6. Dispositif selon la revendication 5, comportant quatre projections radiales (43) et quatre montants axiaux (26), chaque projection radiale (43) coopérant avec un montant axial (26) respectif pour bloquer le déplacement axial dudit poussoir (20) en position de verrouillage de ladite bague de verrouillage (40).

7. Dispositif selon l'une quelconque des revendications précédentes, dans lequel ladite bague de verrouillage (40) comporte une lame axialement flexible (42) pourvue d'une dent (420) saillante axialement vers le haut.

8. Dispositif selon la revendication 7, dans lequel ledit corps interne (10), ou un élément solidaire dudit corps interne (10), tel qu'un couvercle (30), comporte un profil interne (35), comportant une première fenêtre (350) et une seconde fenêtre (351), séparée de ladite première fenêtre (350) par un montant radial (352).

9. Dispositif selon la revendication 8, dans lequel ledit poussoir (20) comporte un doigt axial (27) s'étendant axialement vers le bas.

10. Dispositif selon la revendication 9, dans lequel, en position de verrouillage de ladite bague de verrouillage (40), ladite dent (420) est disposée dans ladite première fenêtre (350), le déplacement de ladite bague de verrouillage (40) vers sa position de libération déformant axialement ladite lame flexible (42) pour permettre à ladite dent (420) de passer dans la seconde fenêtre (351), derrière ledit montant radial (352), ledit montant radial (352) coopérant alors avec ladite dent (420) pour empêcher ladite bague de verrouillage (40) de revenir en position de verrouillage sous l'effet dudit ressort (60).

11. Dispositif selon la revendication 10, dans lequel, lors du déplacement axial dudit poussoir (20) de sa position de repos vers sa position d'actionnement, ledit doigt axial (27) coopère avec ladite dent (420) en la poussant axialement vers le bas pour la désengager dudit montant radial (352), permettant ainsi à ladite bague de verrouillage (40) de revenir vers sa position de verrouillage.

12. Dispositif selon la revendication 11, dans lequel tant que ledit poussoir (20) est déplacé hors de sa position de repos, ledit poussoir (20) bloque ladite bague de verrouillage (40) dans une position intermédiaire entre sa position de libération et sa position de verrouillage.

13. Dispositif selon l'une quelconque des revendications précédentes, dans lequel, en fin d'actionnement, lorsque ledit poussoir (20) revient dans sa position de repos, ladite bague de verrouillage (40) revient automatiquement dans sa position de verrouillage sous l'effet dudit ressort (60).

14. Dispositif selon l'une quelconque des revendications précédentes, dans lequel ledit module de commande électronique (90) comporte des moyens de temporisation pour empêcher après chaque actionnement du distributeur (1) le déplacement de ladite bague de verrouillage (40) de sa position de verrouillage vers sa position de libération pendant un temps prédéterminable.

15. Dispositif selon la revendication 14, dans lequel lesdits moyens de temporisation bloquent un bouton de commande (85) du dispositif et/ou ledit moteur (80).

## Patentansprüche

1. Vorrichtung zur Abgabe eines Fluidprodukts, umfassend:
- einen Fluidproduktspender (1), der ein Fluidprodukt enthaltendes Reservoir (2) und ein Abgabeorgan (3), wie eine Pumpe oder ein Ventil, umfasst, das an dem Reservoir (2) angebracht ist,
- einen Innenkörper (10) mit einem Hohlzylinder (11), der den Spender (1) aufnimmt,
- einen mit einer Abgabeöffnung (21) versehenen Drücker (20), der an dem Innenkörper (10) axial verschiebbar zwischen einer Ruhestellung und einer Betätigungsstellung gelagert ist, wobei die axiale Verschiebung des Drückers (20) von seiner Ruhestellung in seine Betätigungsstellung das Abgabeorgan (3) betätigt, um eine Fluidproduktdosis durch die Abgabeöffnung (21) abzugeben,
**dadurch gekennzeichnet, dass** die Vorrichtung umfasst:
- einen Motor (80),
- ein elektronisches Steuermodul (90),
- ein Zahnrad (50), das mit dem Motor (80) verbunden ist und von dem Motor (80) drehend angetrieben wird, und
- einen Verriegelungsring (40), der zwischen einer Verriegelungsstellung und einer Freigabestellung drehbar an dem Innenkörper (10) gelagert ist, wobei eine Feder (60) den Verriegelungsring (40) in Richtung seiner Verriegelungsstellung beaufschlagt und der Verriegelungsring (40) durch das Zahnrad (50) aus seiner Verriegelungsstellung in seine Freigabestellung bewegt wird, wobei der Verriegelungsring (40) in der Verriegelungsstellung mit dem Drücker (20) zusammenwirkt, um dessen axiale Verschiebung zu verhindern, und in der Freigabestellung mit dem Drücker (20) zusammenwirkt, um dessen axiale Verschiebung in seine Betätigungsstellung zu gestatten.

2. Vorrichtung nach Anspruch 1, enthaltend einen Außenkörper (120), der an dem Innenkörper (10) befestigt ist und den Motor (80) und das elektronische Steuermodul (90) enthält.

3. Vorrichtung nach Anspruch 1 oder 2, enthaltend einen Bedienknopf (85), um die Betätigung des Motors (80) auszulösen.

4. Vorrichtung nach einem der vorhergehenden Ansprüche,
wobei der Drücker (20) zumindest eine axiale Stütze (26) aufweist und der Verriegelungsring (40) zumindest einen radialen Vorsprung (43) aufweist.

5. Vorrichtung nach Anspruch 4,
wobei in der Verriegelungsstellung des Verriegelungsrings (40) der zumindest eine radiale Vorsprung (43) mit der zumindest einen axialen Stütze (26) zusammenwirkt, um die axiale Verschiebung des Drückers (20) in seine Betätigungsstellung zu verhindern, und in der Freigabestellung des Verriegelungsrings (40) der zumindest eine radiale Vorsprung (43) winkelmäßig von der zumindest einen axialen Stütze (26) versetzt ist, um dadurch eine axiale Verschiebung des Drückers (20) in seine Betätigungsstellung zu gestatten.

6. Vorrichtung nach Anspruch 5, enthaltend vier radiale Vorsprünge (43) und vier axiale Stützen (26), wobei jeder radiale Vorsprung (43) mit einer jeweiligen axialen Stütze (26) zusammenwirkt, um die axiale Verschiebung des Drückers (20) in der Verriegelungsstellung des Verriegelungsrings (40) zu sperren.

7. Vorrichtung nach einem der vorhergehenden Ansprüche,
wobei der Verriegelungsring (40) einen axial flexiblen Steg (42) aufweist, der mit einem axial nach oben vorstehenden Zahn (420) versehen ist.

8. Vorrichtung nach Anspruch 7,
wobei der Innenkörper (10) oder ein mit dem Innenkörper (10) fest verbundenes Element, wie ein Deckel (30), ein Innenprofil (35) aufweist, das ein erstes Fenster (350) und ein zweites Fenster (351) umfasst, das von dem ersten Fenster (350) durch eine radiale Stütze (352) getrennt ist.

9. Vorrichtung nach Anspruch 8,
wobei der Drücker (20) einen axialen Finger (27) aufweist, der sich axial nach unten erstreckt.

10. Vorrichtung nach Anspruch 9,
wobei in der Verriegelungsstellung des Verriegelungsrings (40) der Zahn (420) in dem ersten Fenster (350) angeordnet ist, wobei die Bewegung des Verriegelungsrings (40) in seine Freigabestellung den flexiblen Steg (42) axial verformt, um zu ermöglichen, dass der Zahn (420) hinter der radialen Stütze (352) in das zweite Fenster (351) gelangen kann, wobei die radiale Stütze (352) dann mit dem Zahn (420) zusammenwirkt, um zu verhindern, dass der Verriegelungsring (40) unter der Wirkung der Feder (60) in die Verriegelungsstellung zurückkehrt.

11. Vorrichtung nach Anspruch 10,
wobei bei der axialen Verschiebung des Drückers (20) aus seiner Ruhestellung in seine Betätigungsstellung der axiale Finger (27) mit dem Zahn (420) zusammenwirkt, indem er ihn axial nach unten drückt, um ihn aus dem Eingriff mit der radialen Stütze (352) zu lösen, um dadurch dem Verriegelungsring (40) zu gestatten, in seine Verriegelungsstellung zurückzukehren.

12. Vorrichtung nach Anspruch 11,
wobei der Drücker (20) den Verriegelungsring (40) in einer Zwischenstellung zwischen seiner Freigabestellung und seiner Verriegelungsstellung sperrt, solange der Drücker (20) aus seiner Ruhestellung verschoben ist.

13. Vorrichtung nach einem der vorhergehenden Ansprüche,
wobei am Ende der Betätigung, wenn der Drücker (20) in seine Ruhestellung zurückkehrt, der Verriegelungsring (40) unter der Wirkung der Feder (60) selbsttätig in seine Verriegelungsstellung zurückkehrt.

14. Vorrichtung nach einem der vorhergehenden Ansprüche,
wobei das elektronische Steuermodul (90) Verzögerungsmittel enthält, um nach jeder Betätigung des Spenders (1) die Bewegung des Verriegelungsrings (40) aus seiner Verriegelungsstellung in seine Freigabestellung für eine vorbestimmbare Zeit zu verhindern.

15. Vorrichtung nach Anspruch 14,
wobei die Verzögerungsmittel einen Bedienknopf (85) der Vorrichtung und/oder den Motor (80) sperren.

## Claims

1. A device for dispensing a fluid product, comprising:
- a fluid product dispenser (1) comprising a reservoir (2) containing fluid product and a dispensing means (3), such as a pump or a valve, mounted on said reservoir (2),
- an internal body (10) comprising a hollow cylinder (11) receiving said dispenser (1),
- an actuator (20) provided with a dispensing orifice (21), mounted in order to be axially displaceable on said internal body (10) between a rest position and an actuating position, the axial displacement of said actuator (20) from its rest position to its actuating position actuating said dispensing means (3) in order to dispense a dose of fluid product through said dispensing orifice (21),
**characterized in that** said device comprises:
- a motor (80),
- an electronic control module (90),
- a gear wheel (50) connected to said motor (80) and driven in rotation by said motor (80), and
- a locking ring (40) which is rotatably mounted on said internal body (10) between a locking position and a release position, a spring (60) urging said locking ring (40) towards its locking position, said locking ring (40) being displaced from its locking position towards its release position by said gear wheel (50), said locking ring (40) cooperating in the locking position with said actuator (20) in order to prevent its axial displacement and cooperating in the release position with said actuator (20) in order to enable its axial displacement towards its actuating position.

2. The device as claimed in claim 1, comprising an external body (120) fixed to said internal body (10) and containing said motor (80) and said electronic control module (90).

3. The device as claimed in claim 1 or claim 2, comprising a control button (85) for initiating the actuation of said motor (80).

4. The device as claimed in any one of the preceding claims, in which said actuator (20) comprises at least one axial strut (26) and said locking ring (40) comprises at least one radial projection (43).

5. The device as claimed in claim 4 in which, in the locking position of said locking ring (40), said at least one radial projection (43) cooperates with said at least one axial strut (26) in order to prevent the axial displacement of said actuator (20) towards its actuating position, and in the release position of said locking ring (40), said at least one radial projection (43) is angularly offset from said at least one axial strut (26), thereby enabling said actuator (20) to be displaced axially towards its actuating position.

6. The device as claimed in claim 5, comprising four radial projections (43) and four axial struts (26), each radial projection (43) cooperating with a respective axial strut (26) in order to immobilize the axial displacement of said actuator (20) in the locking position of said locking ring (40).

7. The device as claimed in any one of the preceding claims, in which said locking ring (40) comprises an axially flexible tongue (42) provided with an axially upwardly projecting tooth (420).

8. The device as claimed in claim 7, in which said internal body (10), or an element integral with said internal body (10) such as a cover (30), comprises an internal profile (35) comprising a first window (350) and a second window (351) which is separated from said first window (350) by a radial strut (352).

9. The device as claimed in claim 8, in which said actuator (20) comprises an axially downwardly extending axial finger (27).

10. The device as claimed in claim 9 in which, in the locking position of said locking ring (40), said tooth (420) is disposed in said first window (350), the displacement of said locking ring (40) towards its release position axially deforming said flexible tongue (42) in order to enable said tooth (420) to pass into the second window (351) behind said radial strut (352), said radial strut (352) then cooperating with said tooth (420) in order to prevent said locking ring (40) from returning to the locking position under the effect of said spring (60).

11. The device as claimed in claim 10 in which, during the axial displacement of said actuator (20) from its rest position towards its actuating position, said axial finger (27) cooperates with said tooth (420) by pushing it axially downwardly in order to disengage it from said radial strut (352), thereby enabling said locking ring (40) to return towards its locking position.

12. The device as claimed in claim 11 in which, while said actuator (20) is displaced from its rest position, said actuator (20) immobilizes said locking ring (40) in an intermediate position between its release position and its locking position.

13. The device as claimed in any one of the preceding claims in which, at the end of actuation, when said actuator (20) returns to its rest position, said locking ring (40) automatically returns to its locking position under the effect of said spring (60).

14. The device as claimed in any one of the preceding claims, in which said electronic control module (90) comprises time delay means in order to prevent the displacement of said locking ring (40) from its locking position towards its release position for a predeterminable time after each actuation of the dispenser (1).

15. The device as claimed in claim 14, in which said time delay means immobilizes a control button (85) of the device and/or said motor (80).
